# FASCICULE DE BREVET EUROPEEN

(11) **EP 0 621 030 B1**
(45) Date de publication et mention de la délivrance du brevet: **12.08.1998**
(21) Numéro de dépôt: 94400832.5
(22) Date de dépôt: 15.04.1994
(51) Int. Cl.: A61K 7/48

(54) **Utilisation de dérivés de la benzomorpholine dans des compositions cosmétiques ou dermopharmaceutiques à action dépigmentante**
Verwendung von Benzomorpholin-Derivaten in kosmetischen oder dermopharmaceutischen Zusammensetzungen zur Hautdepigmentierung
Use of benzomorpholine derivatives in skin whitening cosmetic or dermopharmaceutical compositions

(30) Priorité: 23.04.1993 FR 9304836
(43) Date de publication de la demande: 26.10.1994
(73) Titulaire: L'OREAL, 75008 Paris (FR)
(72) Inventeur: Lagrange, Alain, F-77700 Coupvray (FR)
(74) Mandataire: Stalla-Bourdillon, Bernard

(56) Documents cités:
- GB-A- 1 245 524

## Description

La présente invention a pour objet l'utilisation de dérivés de la benzomorpholine ou 3,4 dihydro-2H-1,4 benzoxazine dans des compositions cosmétiques ou dermatologiques par application topique dans le but de blanchir la peau ou de traiter les taches pigmentaires.

On rappellera que le mécanisme de formation de la pigmentation de la peau, c'est-à-dire de la formation des mélanines est particulièrement complexe et fait intervenir schématiquement les principales étapes suivantes:
Tyrosine ---> Dopa ---> Dopaquinone ---> Dopachrome ---> Mélanines
l'enzyme intervenant dans cette suite de réactions étant essentiellement la tyrosinase.

Les substances les plus utilisées à l'heure actuelle en tant que dépigmentants sont plus particulièrement l'hydroquinone et ses dérivés en particulier ses éthers tels que le monométhyléther d'hydroquinone.

Ces composés, s'ils ont une efficacité certaine, ne sont malheureusement pas exempts d'effets secondaires, ce qui peut rendre leur emploi délicat voire dangereux.

Ainsi l'hydroquinone, dont l'emploi est d'ailleurs limité à une concentration de 2 %, est un composé particulièrement irritant et cytotoxique pour le mélanocyte, dont le remplacement, total ou partiel a été envisagé par de nombreux auteurs.

Il a ainsi été proposé dans le brevet US n° 4 526 179 certains esters gras d'hydroquinone ayant une bonne activité et étant moins irritants et plus stables que l'hydroquinone.

De même on a proposé d'autres dérivés d'hydroquinone dans la demande japonaise n° 27909/86 ne présentant pas les inconvénients de l'hydroquinone mais dont l'efficacité s'est révélée relativement médiocre.

Il est bien établi qu'une substance exerce une action dépigmentante si elle agit directement sur la vitalité des mélanocytes épidermiques où se déroule normalement la mélanogénèse, et/ou si elle interfère avec une des étapes de la biosynthèse des mélanines soit en inhibant un des enzymes impliqués soit en s'intercalant comme analogue structural dans la voie de synthèse qui peut ainsi être bloquée d'où l'effet dépigmentant.

L'utilisation de substances dépigmentantes topiques présentant une bonne efficacité et étant inoffensives, est tout particulièrement recherchée en vue de traiter les hyperpigmentations régionales par hyperactivité mélanocytaire telles que les mélasmas idiopathiques, survenant lors de la grossesse ("masque de grossesse" ou chloasma) ou secondaires à la contraception oestro-progestative, les hyperpigmentations localisées par hyperactivité et prolifération mélanocytaire bénigne telles que les taches pigmentaires séniles dites lentigo actiniques, les hyperpigmentations accidentelles telles que la photosensibilisation et la cicatrisation post-lésionnelle, ainsi que certaines leucodermies telles que le vitiligo où, à défaut de pouvoir repigmenter la peau lésée, on achève de dépigmenter les zones de peau normale résiduelle pour donner à l'ensemble de la peau une teinte blanche homogène.

Après de nombreuses études sur différentes substances on a constaté de façon tout à fait surprenante que certaines hydroxybenzomorpholines avaient une action dépigmentante, qui pour la plupart, s'est avérée être supérieure à celle de l'hydroquinone dans le test d'inhibition "in vitro" de l'activité de la tyrosinase comme ceci sera décrit ci-après.

La présente invention a donc pour objet l'utilisation, à titre de composés actifs, d'hydroxybenzomorpholines pour la préparation d'une composition cosmétique ou dermatologique ayant une action dépigmentante, lesdits composés répondant à la formule générale suivante : 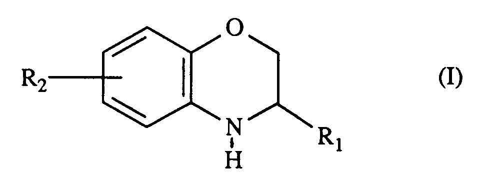 dans laquelle :
R₁ représente un atome d'hydrogène ou un radical alkyle ayant de 1 à 4 atomes de carbone, et
R₂ représente un radical hydroxyle en position 6 ou 7.

Parmi les composés de formule (I), on peut citer notamment les composés suivants :
- la 6-hydroxybenzomorpholine,
- la 7-hydroxybenzomorpholine,
- la 3-méthyl 7-hydroxybenzomorpholine.

Dans les compositions dépigmentantes selon l'invention, la concentration en composés de formule (I) est généralement comprise entre 0,01 % et 10 % et de préférence entre 0,5 % et 5 % en poids par rapport au poids total de la composition.

Le véhicule des compositions peut être notamment une solution aqueuse ou hydroalcoolique, une émulsion du type huile-dans-l'eau ou eau-dans-l'huile, un gel émulsionné ou encore un système biphasé.

De préférence les compositions selon l'invention se présentent sous forme d'une lotion, d'une crème, d'un lait, d'un gel, d'un masque, de microsphères ou nanosphères ou de dispersions vésiculaires. Dans le cas des dispersions vésiculaires, les lipides constitutifs des vésicules peuvent être du type ionique ou non ionique ou bien un mélange de ceux-ci.

Ces compositions cosmétiques peuvent également contenir un humectant, un agent de surface, un kératolytique, un agent anti-inflammatoire, un agent complexant, un anti-oxydant, un conservateur, un parfum ou un filtre solaire.

Ces compositions sont appliquées par voie topique en quantité correspondant aux doses d'application usuelles pour le type de composition considéré (gel, crème, lotion, etc...). Par exemple dans le cas d'une crème on utilise de 0,5 à 3 mg et notamment de 1 à 2 mg de crème par cm2 de peau et par application, à raison d'une ou deux applications par jour.

Les composés de formule générale (I) sont connus et sont obtenus selon les méthodes de synthèse conventionnelles.

### Etudes "in vitro"

Certains des composés de formule générale (I) ont été étudiés comparativement à l'hydroquinone à quantité molaire équivalente dans le test d'inhibition in vitro de l'activité de la tyrosinase.

Selon ce test on suit par spectrométrie visible à 475 nm la quantité de dopachrome formée au cours de la chaîne de réactions de transformation de la tyrosine en mélanines. Ces réactions sont catalysées in vitro par la tyrosinase de champignons, en présence d'un co-substrat réducteur (par exemple la L-dopa en petite quantité) pour initier la réaction d'hydroxylation de la L-tyrosine en L-dopa, laquelle est ensuite oxydée catalytiquement en dopaquinone puis en dopachrome, produit intermédiaire avant les réactions d'oxydation non enzymatiques aboutissant à la formation des mélanines.

On mesure donc la concentration en dopachrome formé au cours du temps en présence et en absence de l'inhibiteur.

Les concentrations en inhibiteurs sont fixées à 50 % molaire par rapport à la concentration en tyrosine dans le milieu réactionnel.

On exprime l'effet d'inhibition par l'abaissement de la quantité maximale de dopachrome formée (valeur de densité optique à 475 nm lue au plateau de la courbe) par rapport à la quantité obtenue en l'absence d'inhibiteur.

### Protocole expérimental

### Réactifs:

A - Tampon phosphate 0,1 M pH = 6,5 (Tween 20 à 1 %)
B - Solution mère de L-tyrosine à 2.10⁻³ M dans A
C - Solution mère de L-dopa à 10⁻⁴ M dans A
D - Solution mère de tyrosinase de champignons à 2.400 unités/ml dans A
E - Solution mère de l'inhibiteur à 10⁻² M dans A (les solutions C et D sont à préparer le jour même)

### Résultats:

- - cuve de référence :: 3 ml de A
- - cuve d'essai :: 1 ml de B
0,1 ml de C
1,85ml de A + E

- homogénéiser et équilibrer à 25°C
- ajouter 0,05 ml de D
- mélanger rapidement et observer la cinétique par la mesure de l'absorbance à 475 nm en fonction du temps.

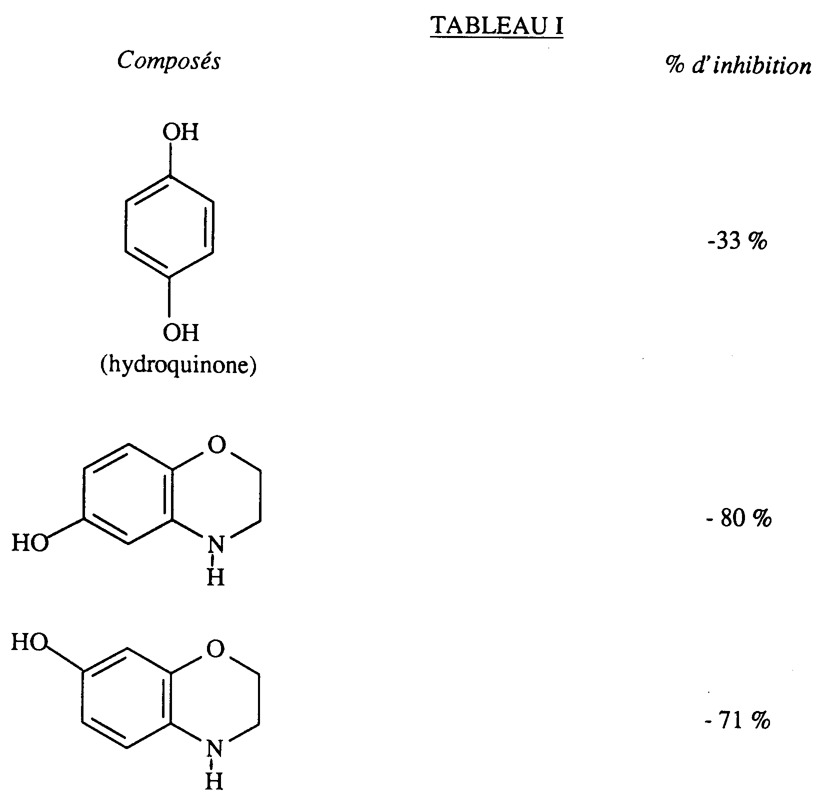

### EXEMPLES DE COMPOSITIONS

### EXEMPLE 1 : Crème dépigmentante

| | |
|---|---|
| - 6-hydroxybenzomorpholine | 1,0 g |
| - Alcool cétylstéarylique oxyéthyléné à 20 moles d'oxyde d'éthylène | 1,0 g |
| - Monostéarate de glycol | 3,0 g |
| - Mélange de caprylate et caprate de coprah | 5,0 g |
| - Polymère d'acide acrylique réticulé vendu sous la dénomination Carbomer 934P par la Société Goodrich | 0,3 g |
| - Triéthanolamine | 0,9 g |
| - Ethanol | 20 g |
| - Glycérine | 3,0 g |
| - Parfum, conservateurs q.s. | |
| - Eau q.s.p. | 100 g |

### EXEMPLE 2 :Lotion dépigmentante

| | |
|---|---|
| - 6-hydroxybenzomorpholine | 2,7 g |
| - Ethanol | 50 g |
| - Polyéthylèneglycol 400 | 30 g |
| - Ethoxy diglycol | 5,0 g |
| - Glycérine | 5,0 g |
| - Eau q.s.p. | 100 g |

### EXEMPLE 3 : Crème dépigmentante

| | |
|---|---|
| - 6-hydroxybenzomorpholine | 0,5 g |
| - Propylèneglycol | 10 g |
| - Diméthylpolysiloxane cyclique | 20 g |
| - Mélange de polycétyldiméthylsiloxane oxyéthyléné et oxypropyléné, d'isostéarate de polyglycéryle à 4 moles de glycérol et de laurate d'hexyle vendu sous la dénomination Abil W 09 par la Société Goldschmidt | 3,0 g |
| - Glycérine | 5,0 g |
| - Parfum, conservateurs q.s. | |
| - Eau q.s.p. | 100 g |

Dans cet exemple, la 6-hydroxybenzomorpholine peut être remplacée par 1,0 g de 7-hydroxybenzomorpholine.

## Revendications

1. Utilisation, à titre de composés actifs, de dérivés de la benzomorpholine pour la préparation d'une composition cosmétique ou dermatologique ayant une action dépigmentante, caractérisée par le fait que lesdits composés répondent à la formule suivante : 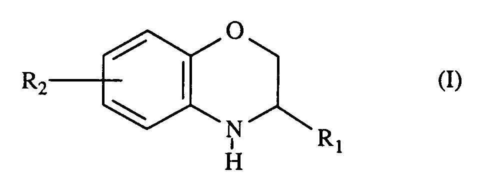 dans laquelle :
R₁ représente un atome d'hydrogène ou un radical alkyle ayant de 1 à 4 atomes de carbone, et
R₂ représente un radical hydroxyle en position 6 ou 7.

2. Utilisation selon la revendication 1, caractérisée par le fait que lesdits composés de formule (I) sont choisis de préférence parmi :
- la 6-hydroxybenzomorpholine,
- la 7-hydroxybenzomorpholine,
- la 3-méthyl 7-hydroxybenzomorpholine.

3. Utilisation selon l'une quelconque des revendications précédentes, caractérisée par le fait que la concentration en composés de formule (I) dans la composition est comprise entre 0,01 % et 10 % en poids par rapport au poids total de la composition.

4. Utilisation selon l'une quelconque des revendications précédentes, caractérisée par le fait que la concentration en composés de formule (I) dans la composition est de préférence comprise entre 0,5 % et 5 % en poids par rapport au poids total de la composition.

5. Utilisation selon l'une quelconque des revendications précédentes caractérisée par le fait que la composition cosmétique ou dermatologique se présente sous forme d'une lotion, d'une crème, d'un lait, d'un gel, d'un masque, de microsphères ou nanosphères ou de dispersions vésiculaires.

6. Utilisation selon l'une quelconque des revendications précédentes, caractérisée par le fait que la composition cosmétique ou dermatologique contient en outre un humectant, un agent de surface, un kératolytique, un agent anti-inflammatoire, un agent complexant, un anti-oxydant, un conservateur, un parfum ou un filtre solaire.

## Claims

1. Use, as active compounds, of benzomorpholine derivatives in the preparation of a cosmetic or dermatological composition having a depigmenting action, characterized in that the said compounds correspond to the following formula: 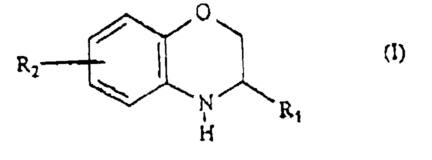 in which:
R₁ represents a hydrogen atom or an alkyl radical having from 1 to 4 carbon atoms, and
R₂ represents a hydroxyl radical in the 6- or 7-position.

2. Use according to Claim 1, characterized in that the said compounds of formula (I) are preferably chosen from:
- 6-hydroxybenzomorpholine,
- 7-hydroxybenzomorpholine,
- 3-methyl-7-hydroxybenzomorpholine.

3. Use according to either of the preceding claims, characterized in that the concentration of compounds of formula (I) in the composition is between 0.01% and 10% by weight with respect to the total weight of the composition.

4. Use according to any one of the preceding claims, characterized in that the concentration of compounds of formula (I) in the composition is preferably between 0.5% and 5% by weight with respect to the total weight of the composition.

5. Use according to any one of the preceding claims, characterized in that the cosmetic or dermatological composition is provided in the form of a lotion, of a cream, of a milk, of a gel, of a mask, of microspheres or nanospheres or of vesicular dispersions.

6. Use according to any one of the preceding claims, characterized in that the cosmetic or dermatological composition additionally contains a humectant, a surface agent, a keratolytic agent, an anti-inflammatory agent, a complexing agent, an anti-oxidizing agent, a preserving agent, a fragrance or a sunscreen.

## Patentansprüche

1. Verwendung von Benzomorpholinderivaten als aktive Verbindung zur Herstellung einer kosmetischen oder dermatologischen Zusammensetzung mit depigmentierender Wirkung, dadurch gekennzeichnet, daß die Verbindung der folgenden Formel entspricht: 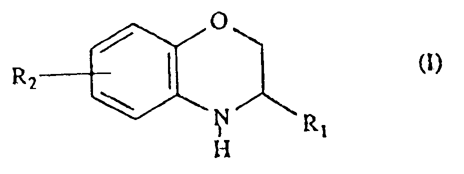 in der:
R₁ ein Wasserstoffatom oder einen Alkylrest mit 1 bis 4 Kohlenstoffatomen, und
R₂ einen Hydroxylrest in den Positionen 6 oder 7
darstellt.

2. Verwendung gemäß Anspruch 1, dadurch gekennzeichnet, daß die Verbindungen der Formel (I) vorzugsweise ausgewählt sind unter:
- dem 6-Hydroxybenzomorpholin,
- dem 7-Hydroxybenzomorpholin, und
- dem 3-Methyl-7-hydroxybenzomorpholin.

3. Verwendung gemäß einem der vorstehenden Ansprüche, dadurch gekennzeichnet, daß die Konzentration der Verbindungen der Formel (I) in der Zubereitung zwischen 0,01 Gew.-% und 10 Gew.-% liegt, bezogen auf das Gesamtgewicht der Zubereitung.

4. Verwendung gemäß einem der vorstehenden Ansprüche, dadurch gekennzeichnet, daß die Konzentration der Verbindungen der Formel (I) in der Zubereitung vorzugsweise zwischen 0,5 Gew.-% und 5 Gew.-% liegt, bezogen auf das Gesamtgewicht der Zubereitung.

5. Verwendung gemäß einem der vorstehenden Ansprüche, dadurch gekennzeichnet, daß die kosmetische oder dermatologische Zubereitung in Form einer Lotion, einer Creme, einer Milch, eines Gels, einer Maske oder in Form einer Dispersion von Mikrokügelchen, Nanokügelchen oder Vesikeln vorliegt.

6. Verwendung gemäß einem der vorstehenden Ansprüche, dadurch gekennzeichnet, daß die kosmetische oder dermatologische Zubereitung zusätzlich ein Feuchthaltemittel, ein oberflächenaktives Mittel, ein Keratolytikum, einen Entzündungshemmer, einen Komplexbildner, ein Antioxidans, ein Konservierungsmittel, ein Parfüm oder ein UV-Filter enthält.
